# EUROPEAN PATENT APPLICATION

(11) **EP 4 209 214 A1**
(43) Date of publication of application: **12.07.2023**
(21) Application number: 21863644.7
(22) Date of filing: 02.09.2021
(51) Int. Cl.: A61K 31/437, A61K 9/107, A61P 17/02

(54) **AK3287 PREPARATION, AND PREPARATION METHOD THEREFOR AND APPLICATION THEREOF**

(30) Priority: 03.09.2020 CN 202010916364
(71) Applicant: Suzhou Ark Biopharmaceutical Co., Ltd, Suzhou, Jiangsu 215123 (CN)
(72) Inventor: PENG, Cheng, Suzhou, Jiangsu 215123 (CN); LI, Yuping, Suzhou, Jiangsu 215123 (CN); ZHOU, Chunyan, Suzhou, Jiangsu 215123 (CN); ZOU, Gang, Suzhou, Jiangsu 215123 (CN); YUAN, Haiqing, Suzhou, Jiangsu 215123 (CN); WU, Zhen Jim, Suzhou, Jiangsu 215123 (CN)
(74) Representative: Algemeen Octrooi- en Merkenbureau B.V.
(86) International application number: PCT/CN2021/116114
(87) International publication number: WO 2022/048580

(57) **Abstract**

An AK3287 preparation, and a preparation method therefor and an application thereof. In mass fraction, the preparation comprises: 0.05-20% of AK3287; 0-30% of an absorption enhancer; 5-99.95% of an oil-phase substance; 0-25% of an emulsifier; 0-5% of a preservative; and 0-70% of water, wherein the structure of AK3287 is shown in the drawing. AK3287 molecules in the preparation have high exposed quantity in the skin, and the preparation can reduce the thickness of the skin at a scar and the ratio of the maximum thickness of the skin at the scar to the thickness of the normal skin; moreover, the relative density of TGF-β and type I collagen in the skin is reduced.

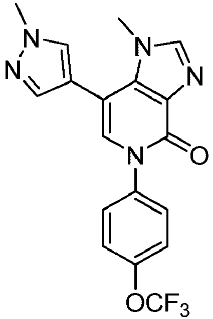

## Description

### CROSS-REFERENCE TO RELATED APPLICATION

The present disclosure claims the priority to and the benefit of Chinese Patent Application No. 202010916364.X, filed on September 3, 2020, entitled "AK3287 PREPARATION, AND PREPARATION METHOD THEREFOR AND APPLICATION THEREOF", the entire contents of which are incorporated herein by reference.

### TECHNICAL FIELD

The present disclosure relates to a pharmaceutical formulation, specifically to an AK3287 formulation for preventing or treating hypertrophic scar, and a preparation method therefor and use thereof.

### BACKGROUND

Fibrosis is a pathogenic hallmark of a range of conditions involving multiple tissues (including liver (*e.g.*, nonalcoholic steatohepatitis, glycogen storage disease type IX, and liver cirrhosis), lung (*e.g.*, chronic interstitial lung disease, pneumoconiosis, silicosis, pulmonary emphysema, fibrotic lung disease, idiopathic pulmonary fibrosis, nonspecific interstitial pneumonia, and cryptogenic organizing pneumonia), vasculature (*e.g.*, diffuse interstitial pulmonary fibrosis and atherosclerosis), heart (*e.g.*, cardiac fibrosis, atrial fibrosis, and endomyocardial fibrosis), skin (*e.g.*, keloid, nephrogenic systemic fibrosis, and scleroderma), joint and interstitial tissue (*e.g.*, joint fibrosis and Dupuytren's disease), pancreas (*e.g*., pancreatitis), mouth (*e.g.*, fibroproliferative lesions in the oral cavity), intestine (*e.g*., Crohn's disease-related fibrotic stricture), and brain (*e.g.*, glial scar and bacterial meningitis-related leptomeningeal fibrosis). Fibrosis may be caused by environmental damages or various injuries, for example, exposure to ionizing radiation (*e.g.*, during cancer treatment), rupture of breast cysts (causing palpable lesions in breast tissues), and excessive deposition of collagen usually due to wounds or tissue damages (*e.g.*, after the injury or surgery). Currently available anti-fibrotic medications include pirfenidone and nintedanib. However, due to the irreversible nature of various fibroses and the limited efficacy of current therapies, there still remains a need for new drugs or other methods for treating such conditions.

Hypertrophic scar (HS) is a serious skin fibrotic disease, which is a pathological process resulting from abnormal wound healing. Hypertrophic scar is mainly formed after healing from the surgery, skin trauma, inflammation, etc., which is one of the common scars. The fibroblasts in hypertrophic scars are abnormally active and proliferate in large quantities, leading to excessive synthesis and deposition of collagen in the extracellular matrix. Migration of fibroblasts causes unbearable itching and pain in the affected part of a subject suffering therefrom. According to incomplete statistics, more than 80 million people are under the influence of the scars every year in the world. In the United States alone, for example, more than 4 million people suffer severe trauma and more than 2 million people suffer burns every year. The incidence of hypertrophic scars after burns is approximately 92%. Hypertrophic scars will cause hump skins, redness, itching, and pain around the injured part of a subject, and even cause tendon contracture or joint dislocation, thereby leading to motor dysfunction and psychological disorders, which seriously affects the subject's appearance, muscle function, and mental health. Hypertrophic scar is one of the most major issues affecting subject's quality of life, and brings heavy burdens to the family and society. At present, various methods have been adopted in the clinic to carry out single or combined therapies including preventive treatments and therapeutic treatments, but the results thereof are still not satisfactory. The preventive treatment mainly includes pressure therapy and flavonoid therapy. When receiving the pressure therapy, the subject is tightened with a bandage around the affected part, which increases the pain of the subject. The flavonoid therapy may cause hormone secretion disorders in the subject's body. The therapeutic treatment includes injection of steroid hormones, surgical excision of scars, and freezing, radiation, and laser therapy. Among these, the hormone therapy will disrupt the subject's hormone secretion; and the surgical excision and the freezing therapy will cause secondary pain to the subject. The therapies described above also have other side effects including pigmentation change, telangiectasia, and local skin atrophy and necrosis. Therefore, there is an urgent need to develop a class of drugs or therapeutic regimens capable of preventing or treating hypertrophic scars.

### SUMMARY

### Technical Problem

In order to solve the above-mentioned technical problem, the present disclosure provides a formulation containing a small-molecule compound, named AK3287, capable of remarkably improving the appearance of scars and reducing the formation of scars, and having a significant efficacy on fibrosis and hypertrophic scars of skin.

### Solution to Problem

In order to solve the above-mentioned technical problem, the present disclosure provides an AK3287 formulation, characterized in that, in mass percentage, the formulation comprises:

| | |
|---|---|
| AK3287 | 0.05-20%; |
| an absorption enhancer | 0-30%; |
| an oil-phase substance | 5-99.95%; |
| an emulsifier | 0-25%; |
| a preservative | 0-5%; and |
| water | 0-70%, wherein AK3287 has the following structure |

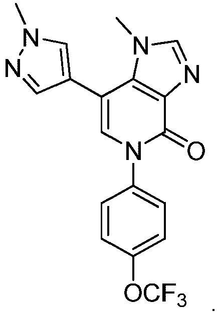

Preferably, the formulation comprises:

| | |
|---|---|
| AK3287 | 0.1-20%; |
| an absorption enhancer | 2-30%; |
| an oil-phase substance | 10-35%; |
| an emulsifier | 5-25%; |
| a preservative | 0.1-5%; and |
| water | 30-70%. |

More preferably, the formulation comprises:

| | |
|---|---|
| AK3287 | 0.5-15%; |
| an absorption enhancer | 5-15%; |
| an oil-phase substance | 10-30%; |
| an emulsifier | 8-20%; |
| a preservative | 0.75-2%; and |
| water | 40-60%. |

Or, more preferably, the formulation comprises:

| | |
|---|---|
| AK3287 | 0.5-15%; |
| an absorption enhancer | 5-15%; |
| an oil-phase substance | 15-30%; |
| an emulsifier | 10-20%; |
| a preservative | 0.2-2%; and |
| water | 40-60%. |

Even more preferably, the formulation comprises:

| | |
|---|---|
| AK3287 | 2-10%; |
| an absorption enhancer | 6-12%; |
| an oil-phase substance | 12-22%; |
| an emulsifier | 10-16%; |
| a preservative | 0.5-1.5%; and |
| water | 45-55%. |

Or, even more preferably, the formulation comprises:

| | |
|---|---|
| AK3287 | 2-8%; |
| an absorption enhancer | 6-10%; |
| an oil-phase substance | 15-25%; |
| an emulsifier | 12-16%; |
| a preservative | 0.2-1%; and |
| water | 45-55%. |

Preferably, the absorption enhancer is selected from the group consisting of diethylene glycol monoethyl ether, dimethyl sulfoxide, and laurocapram. Preferably, the absorption enhancer is diethylene glycol monoethyl ether.

Preferably, the oil-phase substance is one or more of liquid paraffin, octadecanol, hexadecanol, vaseline, silicone, dimethicone, and beeswax. More preferably, the oil-phase substance is a combination of liquid paraffin and octadecanol. The mass ratio of the liquid paraffin to the octadecanol ranges from 1.5:1 to 3:1, preferably 2:1 to 3:1.

Preferably, the emulsifier is one or more of polyethylene glycol-7 stearate, sodium lauryl sulfate, polyoxyethylene (21) stearyl ether, cetearyl glucoside, and polysorbate-80. More preferably, the emulsifier is polyethylene glycol-7 stearate.

Preferably, the preservative is one or more of benzyl alcohol, methylparaben, ethylparaben, benzalkonium chloride, and chlorobutanol. More preferably, the preservative is benzyl alcohol.

Preferably, the formulation is an emulsion.

The present disclosure further provides a method for preparing the AK3287 formulation according to any one of the solutions described above, the method comprising following steps:
(1) weighing water and an absorption enhancer, mixing well, and heating to 60°C to 85°C (preferably 60°C to 70°C); setting a homogenization speed to 1000 rpm to 10000 rpm (preferably 2000 rpm to 6000 rpm); adding AK3287, and homogenizing for 3 min to 10 min to obtain an aqueous solution containing AK3287 drug;
(2) weighing an oil-phase substance and an emulsifier, heating to 60°C to 80°C, and mixing well to obtain an oil-phase solution; and
(3) setting a homogenization speed to 1500 rpm to 5000 rpm (preferably 4000 rpm to 5000 rpm); adding the oil-phase solution obtained from step (2) to the aqueous solution containing drug obtained from step (1) and homogenizing for 5 min to 20 min (preferably 5 min to 10 min); stopping heating; setting a stirring speed to 50 rpm to 500 rpm (preferably 200 rpm to 500 rpm) and stirring continuously; adding a preservative (preferably benzyl alcohol) when the temperature of materials drops to 40°C to 60°C, and keeping stirring until the materials are cooled to room temperature and obtaining the AK3287 formulation.

The present disclosure further provides use of the AK3287 formulation described above in the preparation of a drug for preventing or treating hypertrophic scars or keloids.

### Effects of the Disclosure

The formulation containing the AK3287 small-molecule compound, provided in the present disclosure provides a high exposure of AK3287 to the skin, and is capable of significantly reducing the thickness of the skin at a scar and the ratio of the maximum thickness of the skin at the scar to the thickness of the normal skin, and of remarkably improving the appearance of the scar and reducing the formation of the scar. Meanwhile, the AK3287 formulation of the present disclosure is capable of significantly reducing the relative densities of TGF-β and type I collagen in the skin, having a significant efficacy on fibrosis and hypertrophic scars of skin.

### BRIEF DESCRIPTION OF THE DRAWINGS

FIG. 1 shows a diagram of body weight changes in pigs.
FIG. 2 shows the PK analysis of AK3287 in plasma.
FIG. 3 shows the PK analysis of AK3287 in skin.
FIG. 4 shows the percentage variations in skin thicknesses at the scars in Duroc pigs.
FIG. 5 shows the ratio of the maximum thickness of the skin at the scar to the thickness of the normal skin.
FIG. 6 shows the relative density of TGF-β in skin.
FIG. 7 shows the relative density of type I collagen in skin.

### DETAILED DESCRIPTION

The present disclosure provides an AK3287 formulation, the formulation comprising, in mass percentage,

| | |
|---|---|
| AK3287 | 0.05-20%; |
| an absorption enhancer | 0-30%; |
| an oil-phase substance | 5-99.95%; |
| an emulsifier | 0-25%; |
| a preservative | 0-5%; and |
| water | 0-70%. |

In a preferred embodiment, the formulation comprises, in mass percentage,

| | |
|---|---|
| AK3287 | 0.1-20%; |
| an absorption enhancer | 2-30%; |
| an oil-phase substance | 10-35%; |
| an emulsifier | 5-25%; |
| a preservative | 0.1-5%; and |
| water | 30-70%. |

In a preferred embodiment, the percentage content by mass of AK3287 is 0.05%, 0.1%, 0.2%, 0.5%, 1%, 2%, 4%, 5%, 8%, 10%, 15%, 18%, or 20%, preferably 0.2%, 0.5%, 1%, 2%, 4%, 5%, 8%, 10%, or 15%, more preferably 0.5%, 1%, 2%, 4%, 5%, 8%, or 10%, even more preferably 2%, 4%, 5%, or 8%.

In a preferred embodiment, the absorption enhancer may be diethylene glycol monoethyl ether, dimethyl sulfoxide, or laurocapram, preferably may be diethylene glycol monoethyl ether. The percentage content by mass of the absorption enhancer is 0%, 0.5%, 1%, 2%, 5%, 8%, 10%, 15%, 20%, 25%, or 30%, preferably 2%, 5%, 6%, 8%, 10%, 12%, 15%, or 20%, more preferably 5%, 6%, 8%, 10%, or 12%.

In a preferred embodiment, the oil-phase substance may be one or more of liquid paraffin, octadecanol, hexadecanol, vaseline, silicone, dimethicone, and beeswax. The percentage content by mass of the oil-phase substance is 5%, 10%, 12%, 15%, 18%, 20%, 22%, 25%, 30%, 35%, 50%, 60%, 70%, 80%, 90%, 95%, 99%, 99.9%, or 99.95%, preferably 12%, 15%, 18%, 20%, 22%, 25%, 30%, or 35%, more preferably 12%, 15%, 18%, 22%, or 25%. Among them, the liquid paraffin, also referred to as paraffin oil, is a kind of mineral oil, which is a colorless and odorless mixture obtained from crude oil fractionation, having carbon (C) and hydrogen (H) as the main elements and CₓH_{y} as the chemical formula; the octadecanol is also referred to as octadecan-1-ol or stearyl alcohol. Preferably, the oil-phase substance is a combination of liquid paraffin and octadecanol, wherein the mass ratio of the liquid paraffin to the octadecanol ranges from 1.5:1 to 3:1, preferably 2:1 to 3:1.

The emulsifier may be one or more of polyethylene glycol-7 stearate, sodium lauryl sulfate, polyoxyethylene (21) stearyl ether, cetearyl glucoside, and polysorbate-80, preferably may be polyethylene glycol-7 stearate. The percentage content by mass of the emulsifier is 0%, 1%, 2%, 5%, 8%, 10%, 12%, 14%, 15%, 16%, 18%, 20%, or 25%, preferably 5%, 8%, 10%, 12%, 14%, 15%, 16%, 18%, 20%, or 25%, more preferably 10%, 12%, 14%, 15%, 16%, 18%, or 20%.

In a preferred embodiment, the percentage content by mass of the water is 0%, 10%, 20%, 30%, 40%, 45%, 50%, 51.5%, 53.5%, 55%, 60%, 65%, or 70%, preferably 45%, 50%, 51.5%, 53.5%, 55%, or 60%.

In a preferred embodiment, the preservative may be one or more of benzyl alcohol, methylparaben, ethylparaben, benzalkonium chloride, and chlorobutanol, preferably may be benzyl alcohol. The percentage content by mass of the benzyl alcohol is 0%, 0.1%, 0.2%, 0.5%, 0.75%, 1%, 1.5%, 2%, or 5%, preferably 0.1%, 0.2%, 0.5%, 0.75%, 1%, 1.5%, 2%, or 5%, more preferably 0.75%, 0.2%, 0.5%, 1%, 1.5%, or 2%.

In a preferred embodiment, the formulation is an emulsion.

The present disclosure further provides a method for preparing the AK3287 formulation according to any one of the embodiments described above, the method comprising the steps of:
(1) weighing water and an absorption enhancer, mixing well, and heating to 60°C to 85°C (preferably 60°C to 70°C); setting a homogenization speed to 1000 rpm to 10000 rpm (preferably 2000 rpm to 6000 rpm); adding AK3287, and homogenizing for 3 min to 10 min to obtain an aqueous solution containing drug;
(2) weighing an oil-phase substance and an emulsifier, heating to 60°C to 80°C, and mixing well to obtain an oil-phase solution; and
(3) setting a homogenization speed to 1500 rpm to 5000 rpm (preferably 4000 rpm to 5000 rpm); adding the oil-phase solution obtained from step (2) to the aqueous solution containing drug obtained from step (1) and homogenizing for 5 min to 20 min (preferably 5 min to 10 min); stopping heating; setting a stirring speed to 50 rpm to 500 rpm (preferably 200 rpm to 500 rpm) and stirring continuously; adding a preservative when the temperature of materials drops to 40°C to 60°C, and keeping stirring until the materials are cooled to room temperature and obtaining the AK3287 formulation.

Finally, the present disclosure further provides use of the AK3287 formulation described above in the preparation of a drug for preventing or treating hypertrophic scar or keloid.

The following examples are for illustrative purposes only and are not intended to limit the scope of the claims provided in the present disclosure.

### Examples 1 to 3

Provided was an emulsion containing AK3287 in different proportions, and the specific formulae thereof in Examples 1 to 3 were shown in Table 1 to Table 3 below, respectively.

**Table 1. Formula Information of AK3287 Emulsion (Strength: 8.0%)**

| **Formula Ingredients** | **Function** | **Content % (w/w)** |
|---|---|---|
| AK3287 | Active ingredient | 8.0 |
| Diethylene glycol monoethyl ether | Absorption enhancer | 8.0 |
| Liquid paraffin | Oil-phase | 12.0 |
| Octadecanol | | 6.0 |
| Polyethylene glycol-7 stearate | Emulsifier | 14.0 |
| Benzyl alcohol | Preservative | 0.5 |
| Water | Water-phase | 51.5 |
| Total | / | 100.00 |

**Table 2. Formula Information of AK3287 Emulsion (Strength: 2.0%)**

| **Formula Ingredients** | **Function** | **Content % (w/w)** |
|---|---|---|
| AK3287 | Active ingredient | 2.0 |
| Diethylene glycol monoethyl ether | Absorption enhancer | 8.0 |
| Liquid paraffin | Oil-phase | 16.0 |
| Octadecanol | | 6.0 |
| Polyethylene glycol-7 stearate | Emulsifier | 14.0 |
| Benzyl alcohol | Preservative | 0.5 |
| Water | Water-phase | 53.5 |
| Total | / | 100.00 |

**Table 3. Formula Information of AK3287 Emulsion (Strength: 0.5%)**

| **Formula Ingredient** | **Function** | **Content % (w/w)** |
|---|---|---|
| AK3287 | Active ingredient | 0.5 |
| Diethylene glycol monoethyl ether | Absorption enhancer | 8.0 |
| Liquid paraffin | Oil-phase | 16.0 |
| Octadecanol | | 6.0 |
| Polyethylene glycol-7 stearate | Emulsifier | 14.0 |
| Benzyl alcohol | Preservative | 0.5 |
| Water | Water-phase | 55.0 |
| Total | / | 100.00 |

The preparation method for the AK3287-containing emulsions in Examples 1 to 3 specifically comprised the following steps:
1. preparation of an aqueous solution containing drug: appropriate amounts of water and diethylene glycol monoethyl ether were each weighed according to the proportions in the formulae of Examples 1 to 3, mixed well, and heated at 60°C to 70°C; the homogenization speed was set to 2000 rpm to 6000 rpm and a formulated dose of AK3287 was added while homogenizing for 3 min to 10 min;
2. preparation of an oil-phase solution: formulated doses of liquid paraffin, octadecanol, and polyethylene glycol-7 stearate were each weighed and heated at 70°C; mixed well after dissolution; and
3. formation by emulsification: the homogenization speed was set to 4000 rpm to 5000 rpm; the oil-phase solution was slowly added to the aqueous solution containing drug and homogenized for 5 min to 10 min; the heating was stopped; the stirring speed was set to 300 rpm and the materials were stirred continuously; a formulated dose of benzyl alcohol was added when the temperature of the materials dropped to 50°C; and the materials were kept stirring until the materials were cooled to room temperature.

### Example 4

### Animal model: Duroc pig model of hypertrophic scar

1. Grouping of red Duroc pigs and construction of full-thickness skin excisional wound models:
Twelve female Duroc pigs weighing about 20 kg were divided into 4 groups, and the grouping condition was shown in Table 4 below. After 7 days of adaptive feeding, no obvious abnormalities were observed in all of the Duroc pigs which were sequentially enrolled in the experiment.
Three square wounds , each having an area of 4 cm × 4 cm were made with a dermatome on the left and right sides of the dorsal spine of Duroc pig. The depths of incised wounds by the dermatome on the left and right sides were 0.045 inches and 0.060 inches, respectively. The wounds were spaced from one another by 4 cm or more.

2. Mode and dosage of administration:
According to Table 4, 1 ml of the AK3287 emulsion or vehicle was administered to each of the wounds and smeared evenly on the wounds. The above administration treatment was carried out once daily. The time points for administration were set to start on Day 3 and end on Day 21.

**Table 4. Route, dosage, and regimen of administration to red Duroc pig models**

| No. | Number of Pigs (pcs) | Group | AK3287 Content | Mode of Administration | Timing of Administration |
|---|---|---|---|---|---|
| 1 | 3 | Vehicle | 0 | application on wound | Time points for administration set to start on Day 3 and end on Day 21 |
| 2 | 3 | Test substance (low dose) | 0.5% | application on wound | |
| 3 | 3 | Test substance (medium dose) | 2% | application on wound | |
| 4 | 3 | Test substance (high dose) | 8% | application on wound | |

3. Sample and analysis of PK test:
On Day 4, Day 9, Day 15, and Day 21 (i.e., on Day 1, Day 6, Day 12, and Day 18 after the first administration), a piece of circular skin was obtained by surgical incision with a skin biopsy punch (10 mm), after the animal was anesthetized and before the drug was smeared. Blood was sampled from the jugular vein and prepared as plasma. A total of 48 skin samples and 48 plasma samples were collected and subjected to PK analysis by LC/MS method.
4. Research indicators and sampling methods:
1) The animal was weighed every two weeks.
2) The wound was photographed on Day 0, Day 1, Day 3, Day 5, Day 7, Day 14, Day 21, Day 28, Day 42, Day 56, Day 84, Day 112, and Day 140. The area of the ulcer wound was imitatively measured by software, and the time required for wound healing was recorded.
3) After the animal was euthanized, the skin over the wound was taken. The test skin was placed in formalin and stained with HE to evaluate the wound healing.
4) The expressions of transforming growth factor-β (TGF-β) and type I collagen were evaluated by immunohistochemical staining.

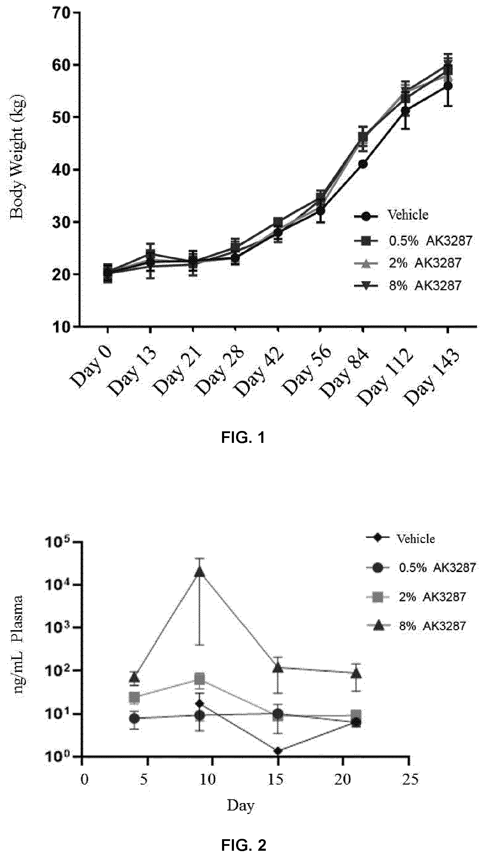

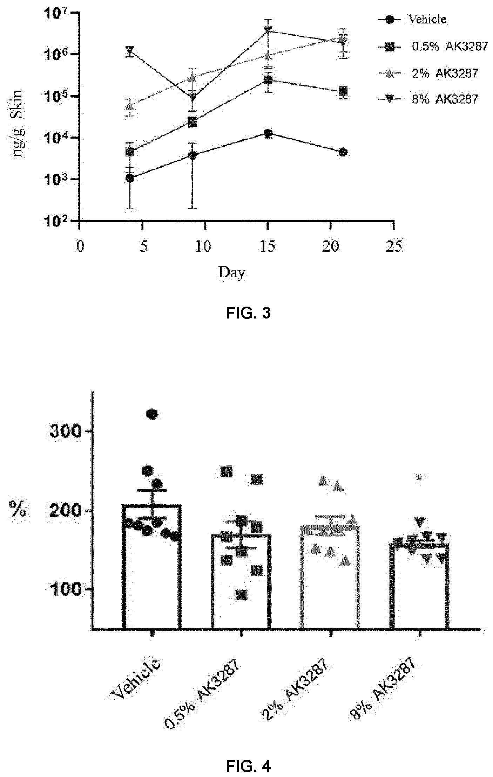

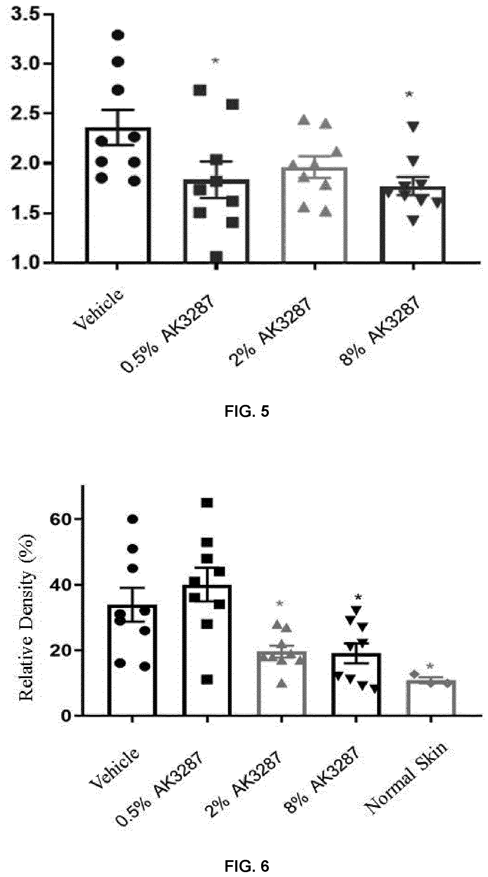

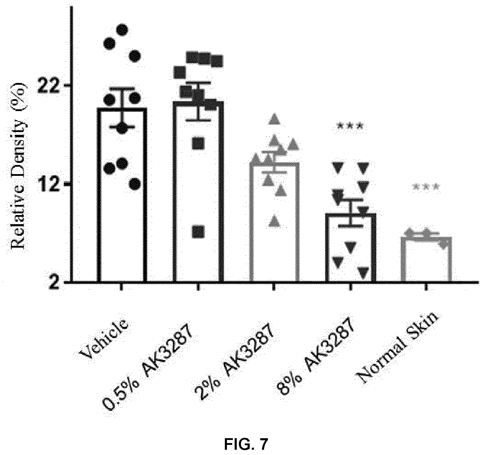

5. Results:
1) There was no significant difference in body weight between all the pigs in the administration groups and the pigs in the vehicle group (FIG. 1), and their behaviors were normal.
2) PK analysis showed that a small amount of AK3287 could enter the blood (FIG. 2), but a majority of AK3287 remained in the skin tissues (FIG. 3), and the exposure in the skin was higher in the high-dose group.
3) For 0.06-inch wounds, compared with the vehicle group, the 8% AK3287 emulsion could significantly reduce the percentage variation in the skin thickness at the scar (FIG. 4) and the ratio of the maximum thickness of the skin at the scar to the thickness of the normal skin by 24.4% and 25.0%, respectively (FIG. 5).
4) For 0.06-inch wounds, compared with the vehicle group, the 8% AK3287 emulsion could significantly reduce the relative densities of TGF-β and type I collagen in skin by 43.5% and 54.1%, respectively (FIG. 6 and FIG. 7).

In all of the drawings noted above, p<0.05 means a significant difference; * indicates p<0.05; ** indicates p<001; and *** indicates p<0.001.

## Claims

1. An AK3287 formulation, **characterized in that**, in mass percentage, the formulation comprises:
| | |
|---|---|
| AK3287 | 0.05-20%; |
| an absorption enhancer | 0-30%; |
| an oil-phase substance | 5-99.95%; |
| an emulsifier | 0-25%; |
| a preservative | 0-5%; and |
| water | 0-70%, wherein AK3287 has the following structure |

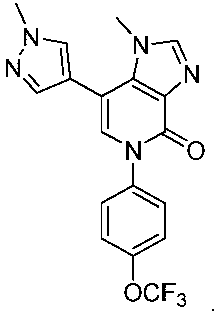

2. The AK3287 formulation according to claim 1, **characterized in that**, the formulation comprises:
| | |
|---|---|
| AK3287 | 0.1-20%; |
| an absorption enhancer | 2-30%; |
| an oil-phase substance | 10-35%; |
| an emulsifier | 5-25%; |
| a preservative | 0.1-5%; and |
| water | 30-70%; |
preferably, the formulation comprises:
| | |
|---|---|
| AK3287 | 0.5-15%; |
| an absorption enhancer | 5-15%; |
| an oil-phase substance | 10-30%; |
| an emulsifier | 8-20%; |
| a preservative | 0.75-2%; and |
| water | 40-60%; |
or, the formulation comprises:
| | |
|---|---|
| AK3287 | 0.5-15%; |
| an absorption enhancer | 5-15%; |
| an oil-phase substance | 15-30%; |
| an emulsifier | 10-20%; |
| a preservative | 0.2-2%; and |
| water | 40-60%. |

3. The AK3287 formulation according to claim 1, **characterized in that**, the formulation comprises:
| | |
|---|---|
| AK3287 | 2-10%; |
| an absorption enhancer | 6-12%; |
| an oil-phase substance | 12-22%; |
| an emulsifier | 10-16%; |
| a preservative | 0.5-1.5%; and |
| water | 45-55%; |
or, the formulation comprises:
| | |
|---|---|
| AK3287 | 2-8%; |
| an absorption enhancer | 6-10%; |
| an oil-phase substance | 15-25%; |
| an emulsifier | 12-16%; |
| a preservative | 0.2-1%; and |
| water | 45-55%. |

4. The AK3287 formulation according to any one of claims 1 to 3, **characterized in that**, the absorption enhancer is selected from the group consisting of diethylene glycol monoethyl ether, dimethyl sulfoxide, and laurocapram, preferably, the absorption enhancer is diethylene glycol monoethyl ether.

5. The AK3287 formulation according to any one of claims 1 to 3, **characterized in that**, the oil-phase substance is one or more of liquid paraffin, octadecanol, hexadecanol, vaseline, silicone, dimethicone, and beeswax; preferably, the oil-phase substance is a combination of liquid paraffin and octadecanol, wherein a mass ratio of the liquid paraffin to the octadecanol ranges from 1.5:1 to 3:1, preferably from 2:1 to 3:1.

6. The AK3287 formulation according to any one of claims 1 to 3, **characterized in that**, the emulsifier is one or more of polyethylene glycol-7 stearate, sodium lauryl sulfate, polyoxyethylene (21) stearyl ether, cetearyl glucoside, and polysorbate-80, preferably, the emulsifier is polyethylene glycol-7 stearate.

7. The AK3287 formulation according to any one of claims 1 to 3, **characterized in that**, the preservative is one or more of benzyl alcohol, methylparaben, ethylparaben, benzalkonium chloride, and chlorobutanol, preferably, the preservative is benzyl alcohol.

8. The AK3287 formulation according to any one of claims 1 to 3, **characterized in that**, the formulation is an emulsion.

9. A method for preparing the AK3287 formulation according to any one of claims 1 to 8, the method comprising following steps:
(1) weighing water and an absorption enhancer, mixing well, and heating to 60°C to 85°C, preferably 60°C to 70°C; setting a homogenization speed to 1000 rpm to 10000 rpm, preferably 2000 rpm to 6000 rpm; adding AK3287, and homogenizing for 3 min to 10 min to obtain an aqueous solution containing drug;
(2) weighing an oil-phase substance and an emulsifier, heating to 60°C to 80°C, and mixing well to obtain an oil-phase solution; and
(3) setting a homogenization speed to 1500 rpm to 5000 rpm, preferably 4000 rpm to 5000 rpm; adding the oil-phase solution obtained from step (2) to the aqueous solution containing drug obtained from step (1) and homogenizing for 5 min to 20 min, preferably 5 min to 10 min; stopping heating; setting a stirring speed to 50 rpm to 500 rpm, preferably 200 rpm to 500 rpm and stirring continuously; adding a preservative when the temperature of materials drops to 40°C to 60°C, and keeping stirring until the materials are cooled to room temperature and obtaining the AK3287 formulation.

10. Use of the AK3287 formulation according to any one of claims 1 to 8 in the preparation of a drug for preventing or treating hypertrophic scars or keloids.
